# EUROPÄISCHE PATENTSCHRIFT

(11) **EP 1 841 732 B1**
(45) Veröffentlichungstag und Bekanntmachung des Hinweises auf die Patenterteilung: **23.12.2009**
(21) Anmeldenummer: 06700232.9
(22) Anmeldetag: 10.01.2006
(51) Int. Cl.: C07C 319/08

(54) **HERSTELLUNG VON 3-(ALKYLTHIO)PROPANAL**
PRODUCTION OF 3-(ALKYLTHIO)PROPANAL
PRODUCTION DE 3-(ALKYLTHIO)PROPANAL

(30) Priorität: 28.01.2005 DE 102005003990
(43) Veröffentlichungstag der Anmeldung: 10.10.2007
(73) Patentinhaber: Evonik Degussa GmbH, 45128 Essen (DE)
(72) Erfinder: REDLINGSHÖFER, Hubert, 91481 Münchsteinach (DE); WECKBECKER, Christoph, 63584 Gründau-Lieblos (DE); HUTHMACHER, Klaus, 63571 Gelnhausen (DE); FISCHER, Achim, 63739 Aschaffenburg (DE); BARTH, Jan-olaf, 60598 Frankfurt (DE)
(86) Internationale Anmeldenummer: PCT/EP2006/050132
(87) Internationale Veröffentlichungsnummer: WO 2006/079582

(56) Entgegenhaltungen:
- DE-C1- 4 238 493
- US-A- 5 705 675
- US-A- 5 775 329

## Beschreibung

Die Erfindung betrifft ein Verfahren zur Herstellung von 3-(Alkylthio)propanal aus Glycerin unter Verwendung von Katalysatoren.

Neben dem industriell wichtigen MMP können auch MMP-Analoge der allgemeinen Form 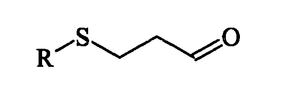 mit R = H, Alkyl aus Glycerin hergestellt werden.

3-(Methylthio)propanal (MMP) ist ein wichtiges Zwischenprodukt und somit von großer wirtschaftlicher Bedeutung für die Darstellung von D,L-Methionin und dem Methionin-Hydroxy-Analogen 2-Hydroxy-4-methylthiobuttersäure (MHA). Methionin ist eine essentielle Aminosäure, die u.a. als Ergänzung in Futtermitteln eingesetzt wird. Nutritivitätsverbessernde Futtermittelzusatzstoffe sind heute ein unverzichtbarer Bestandteil der Tierernährung. Sie dienen der besseren Verwertung des Nahrungsangebotes, stimulieren das Wachstum und fördern die Eiweißbildung. Einer der wichtigsten dieser Zusatzstoffe ist die essentielle Aminosäure Methionin, die vor allem in der Geflügelaufzucht als Futtermitteladditiv eine herausragende Stellung einnimmt. Auf diesem Gebiet haben, aber auch sogenannte Methionin-Ersatzstoffe wie das Methionin-Hydroxy-Analog (abgekürzt MHA) nicht unerhebliche Bedeutung, da sie ähnliche wachstumsstimulierende Eigenschaften aufweisen wie die dafür bekannte Aminosäure.

MMP wird nach dem Stand der Technik durch katalysierte Addition von Methylmercaptan an Acrolein hergestellt. In der Regel wird flüssiges Acrolein mit Methylmercaptan in einem Reaktor zur Reaktion gebracht, in dem bereits flüssiges MMP und der Katalysator gelöst vorliegen (DT 2320544). Ebenfalls bekannt ist der Einsatz von gasförmigem Acrolein mit Methylmercaptan (FR 7520183, FR 7917827, WO 97/00858). Die Reaktion zwischen Methylmercaptan und Acrolein kann batchweise oder kontinuierlich durchgeführt werden (US 4,225,515, US 5,352,837). Als konventionelle Katalysatoren dienen organische Basen, z.B. tertiäre Amine wie Hexamethylentetramin, Trialkylamine, z.B. Triethyl- oder Triethanolamin, Benzylamine, Pyridine, z.B. 2-Fluorpyridin und 4-Dimethylaminopyridin, Picolin, Pyrazin, Imidazol und Nicotinamid, aber auch Kupfer-(II)-acetat, Quecksilbermethylmercaptid und organische Peroxide.

Auch die Verwendung von Ionenaustauschern wurde erwähnt (FR 7520183). Üblicherweise wird der eigentliche Additions-Katalysator mit einem Hilfskatalysator, einer organischen Säure, z.B. Essigsäure, Zitronensäure oder Ameisensäure oder einer Mineralsäure, z.B. Schwefel- oder Phosphorsäure kombiniert, um zum einen die Polymerisation von Acrolein, also die Bildung von unerwünschten Nebenprodukten, zu inhibieren und zum anderen die generelle Ausbeute durch Konditionierung der zugesetzten Base zu erhöhen. Der Katalysator wird nicht zurückgewonnen und geht während der Aufarbeitung verloren.

Typische Katalysatorkonzentrationen liegen bei 0,001 bis 0,005 Mol-% bezogen auf Methylmercaptan. Die notwendige Menge an Säure, typischerweise Essigsäure, liegt zwischen 0,5 und 50 Mol-%. Zur Vereinfachung der MMP-Herstellverfahren können Katalysator und Säure in einem Prämix vorgemischt und als Lösung zudosiert werden. Die Konzentration an Katalysator-Prämix im flüssigen MMP-Reaktionsmedium beträgt üblicherweise 0,2 bis 0,75 Gew.%. Nach beendeter Reaktion wird das MMP von den Hilfs- und Nebenprodukten durch Destillation getrennt. Während der destillativen Aufreinigung des so hergestellten Additionsproduktes geht der Katalysator-Prämix verloren und muss je nach Siedepunkt über den Destillationssumpf oder das Abgas entsorgt werden. Grundsätzlich können Teile des Katalysators oder der zugesetzten Säure während der Destillation über Kopf gehen und das gewünschte Rein-MMP verunreinigen.

Nachteilig an diesem Verfahren ist neben dem Verbrauch des Katalysators die mehrstufige Synthese von MMP. So muss das benötigte Zwischenprodukt Acrolein aufwendig durch selektive Oxidation aus Propen in der Gasphase hergestellt und in der mehrstufigen Aufarbeitung isoliert werden.

Nach dem Stand der Technik erfolgt die Synthese von Acrolein durch heterogen katalysierte selektive Oxidation von Propen an Mischoxidkatalysatoren. EP 417723 beschreibt die Synthese an komplexen Multimetallmischoxidkatalysatoren bei Temperaturen von 300 bis 380 °C und Drücken von 1,4 bis 2,2 bar. In Ullmann's Encyclopedia of Industrial Chemistry, 6. Auflage, 1999 ist das gesamte Verfahren inklusive Aufarbeitung, wo mehrere Nebenprodukte abgetrennt werden, beschrieben. Nachdem das Eduktgemisch aus Propen, Luft und Wasser zumindest teilweise am Katalysator umgesetzt wurde, erfolgt zunächst das Quenchen zur Abtrennung hochsiedender Nebenprodukte wie Polymere, Acrylsäure und Essigsäure. Im anschließenden Absorber wird Acrolein ausgewaschen. Nach der Desorption zur Rückgewinnung des Absorptionsmittels wird das erhaltene Rohacrolein mehrstufig destillativ gereinigt.

Bisher wird Glycerin nicht zur Synthese von MMP verwendet. Weiterhin ist die direkte Synthese von MMP aus Glycerin nicht bekannt. Es ist jedoch bekannt, dass sich Glycerin in Gegenwart sauerer Stoffe zu verschiedenen Produkten dehydratisieren lässt.

Gemäß Organic Synthesis I, 15-18 (1964) wird durch Behandeln eines Gemisches aus pulverförmigem Kaliumhydrogensulfat, Kaliumsulfat und Glycerin bei 190 bis 200 °C Acrolein in einer Ausbeute zwischen 33 und 48 % gewonnen. Aufgrund der niedrigen Ausbeuten und der hohen Salzfrachten eignet sich dieses Verfahren jedoch nicht für den technischen Maßstab.

Im Rahmen der Untersuchungen von Modellsubstanzen von Biomasse Pyrolyseölen wurde auch die katalytische Behandlung von Glycerin an H-ZSM5-Zeolithen bei 350 bis 500 °C untersucht - siehe Dao, Le H. et al. ACS Symp. Ser.: 376 (Pyrolysis Oils Biomass) 328-341 (1988). Kohlenwasserstoffe werden nur in geringen Ausbeuten gebildet.

In der DE 42 38 493 ist ferner die säure-katalysierte Umsetzung von Glycerin zu Acrolein in der Gas- und in der Flüssigphase beschrieben. Die DE 42 38 492 betrifft die Synthese von 1,2 und 1,3 Propandiol durch Dehydratisierung von Glycerin mit hohen Ausbeuten.

Für die direkte Synthese von MMP aus Glycerin ist jedoch neben der Beteiligung von Dehydratisierungsschritten die gleichzeitige selektive Einbindung einer schwefelhaltigen Verbindung wie von Methylmercaptan erforderlich.

Erfindungsgemäss wird ein Verfahren insbesondere zur Darstellung von MMP aus Glycerin ohne Isolation von Zwischenprodukten zur Verfügung gestellt, wobei das nach dem Stand der Technik mehrstufige Synthese von MMP jetzt einstufig unter Einsatz eines geeigneten Katalysators durchgeführt werden kann.

Gegenstand der Erfindung ist ein Verfahren zur Herstellung von Verbindungen der allgemeinen Formel 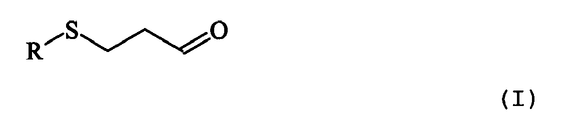 in der bedeuten
R: H, C₁ bis C₃-Alkyl, durch die Umsetzung von Glycerin oder einer Verbindung, aus der Glycerin freigesetzt wird, mit einer Verbindung der allgemeinen Formel

R-SH (II)

in der bedeutet
R: H, C₁ bis C₃-Alkyl
oder Verbindungen, aus denen diese (II) hergestellt wird, in Gegenwart eines sauren Feststoffkatalysators, dessen H₀-Wert (Hammet'sche Säurefunktion) kleiner +2 ist.

Bevorzugtes Produkt ist MMP, das unter Verwendung von Methylmercaptan hergestellt wird.

Dabei wird zum Beispiel ein Glycerin-Methylmercaptan-Gemisch, gegebenenfalls in Gegenwart eines Lösungsmittels entweder in der Flüssigphase oder in der Gasphase an einem sauren Feststoffkatalysator umgesetzt.

Erfolgt die Synthese in der Flüssigphase, wird bei einer Reaktionstemperatur zwischen 50 und 500 °C, bevorzugt zwischen 80 und 3500 °C, besonders bevorzugt zwischen 120 und 300 °C gearbeitet. Der Druck wird dabei derart eingestellt, dass der flüssige Zustand des Reaktionsgemisches erhalten bleibt. Üblicherweise beträgt der Druck zwischen 1 und 300 bar, bevorzugt zwischen 5 und 200 bar, besonders bevorzugt zwischen 20 und 150 bar. In der Flüssigphase kann die Synthese entweder in Gegenwart eines homogenen oder eines bevorzugt heterogenen Katalysators durchgeführt werden.

In der Flüssigphase ist weiterhin die Verwendung eines Lösungsmittels oder Verdünnungsmittels bevorzugt. Dadurch werden die Konzentration an Glycerin gesenkt und Nebenreaktionen zu Oligomeren, Polymeren und anderen Hochsiedern minimiert. Eingesetzt werden dem Fachmann bekannte Lösungs- und Verdünnungsmittel wie beispielsweise Wasser, Alkohole wie zum Beispiel Methanol und Ethanol, Aceton, Toluol oder Methylisobutylketon. Auch MMP selbst kann als Lösungsmittel eingesetzt werden, was den Vorteil hat, dass kein zusätzlicher Stoff eingesetzt wird und somit die Aufarbeitung vereinfacht ist.

Im Reaktionsgemisch beträgt die Glycerinkonzentration zwischen 1 und 100 Gew.-%, bevorzugt zwischen 1 und 70 Gew.-% und insbesondere zwischen 5 und 40 Gew.-%, bezogen auf das Lösungs- oder Verdünnungsmittel. Das molare Verhältnis zwischen Glycerin und Methylmercaptan wird zwischen 0,2 und 50, bevorzugt zwischen 0,4 und 30, insbesondere zwischen 0,8 und 10 eingestellt.

Im allgemeinen ist eine homogene Lösung für einen guten Stoffaustausch vorteilhaft, aber hier nicht zwingend erforderlich. Dies kann sogar durch das Prinzip der ZweiPhasen-Katalyse gezielt ausgenutzt werden, wobei beispielsweise ausschließlich das Produkt MMP in der Lösemittelphase löslich ist. Ist das Reaktionsprodukt MMP unlöslich im Reaktionsmedium, kann das Produkt ohne aufwendige Aufarbeitung durch Phasentrennung vom Reaktionsmedium separiert und somit das Gesamtverfahren vereinfacht werden.

Erfolgt die Synthese in der Gasphase, wird die Reaktion bei einer Temperatur zwischen 200 und 550 °C, bevorzugt zwischen 220 und 450 °C, besonders bevorzugt zwischen 250 und 350 °C durchgeführt. Üblicherweise beträgt der Druck zwischen 1 und 100 bar, bevorzugt zwischen 1 und 70 bar, besonders bevorzugt zwischen 1 und 30 bar. In der Gasphase wird die Synthese in Gegenwart eines Feststoffkatalysators durchgeführt.

Auch in der Gasphase ist die Verwendung eines Verdünnungsmittels bevorzugt. Dadurch wird die Konzentration an Glycerin auf die oben angegebenen Werte gesenkt und somit werden Nebenreaktionen zu Oligomeren, Polymeren und anderen Hochsiedern minimiert. Eingesetzt werden dem Fachmann bekannte Verdünnungsmittel wie beispielsweise Stickstoff, Luft oder Wasser. Bevorzugt werden Verdünnungsmedien, die nach der Kondensation durch Phasentrennung einfach von MMP isoliert werden können.

Unabhängig von der Durchführung in der Gas- oder Flüssigphase wird durch die Verwendung von Glycerin als Rohstoff die Konzentration von reaktiven Intermediaten wie möglicherweise Acrolein, Allylalkohol, Acroleinacetale, 3-Hydroxypropanal oder radikalische sowie ionische Verbindungen mit drei Kohlenstoffatomen durch Weiterreaktion zu MMP auf einem vergleichsweise niedrigen Niveau eingestellt, da diese Intermediate schnell zu MMP weiterreagieren können. Eine hohe Konzentration von reaktiven Intermediaten würde zu vermehrter Bildung hochsiedender Rückstände führen und ist daher nicht wünschenswert. So wird nach dem Stand der Technik beispielsweise aber noch Acrolein in hoher Konzentration aufgearbeitet und als isoliertes Zwischenprodukt eingesetzt.

Weiterhin sind durch die Umwandlung von Glycerin zu dehydratisierten reaktiven Verbindungen, die unter Anwesenheit von Methylmercaptan schnell zu MMP weiterreagieren, sehr hohe Umsätze von Glycerin ohne die Bildung von hochsiedenden Nebenprodukten möglich.

Gebildetes MMP kann dann in bekannter Weise allein oder zusammen mit einem Teil des Lösungs- oder Verdünnungsmediums durch Strippen, Destillation oder Extraktion aus dem Reaktionsgemisch abgetrennt werden. Nicht umgesetztes Glycerin kann dann in die Reaktionsstufe zurückgeführt werden.

Ein weiterer Vorteil des Verfahrens besteht darin, dass auch Glycerinlösungen mit einem Gehalt von 5 bis 40 Gew.-% verwendbar sind. Somit sind sogenannte Rohglycerine ohne vorherige Aufkonzentrierung oder Reinigung direkt für die Synthese von MMP einsetzbar.

Die Durchführung kann in dem Fachmann bekannten Reaktionsgefäßen wie beispielsweise Festbettreaktoren, Rührkesseln, Strömungsrohren oder Blasensäulen erfolgen.

Methylmercaptan kann dabei entweder in flüssiger oder gasförmiger Form eingesetzt werden. Weiterhin kann es als Reinsubstanz oder als Roh-Methylmercaptan mit Verunreinigungen, wie beispielsweise Methanol, Dimethylsulfid, Dimethylpolysulfide, Schwefelwasserstoff oder Dimethylether, verwendet werden. Die Verwendung von Roh-Methylmercaptan hat den Vorteil, dass ein kostengünstigerer Rohstoff, der nicht weiter aufgearbeitet werden muss, einsetzbar ist.

Als saure heterogene Katalysatoren werden im allgemeinen unlösliche Stoffe eingesetzt, die neben der Dehydratisierung von Glycerin gleichzeitig, die Einbindung von Methylmercaptan zu MMP selektiv beschleunigen. Diese weisen bevorzugt einen H₀-Wert kleiner +2, insbesondere kleiner -3 auf. Der H₀-Wert entspricht der Säurefunktion nach Hammett und lässt sich durch die sogenannte Amintitration unter Verwendung von Indikationen oder durch Adsorption einer gasförmigen Base ermitteln - siehe Studies in surface science and catalysis, Vol. 51, 1989: "New solid acids and bases, their catalytic properties", K. Tanabe et al., Kapitel 2, insbesondere Seiten 5-9, Kapitel 1 (Seiten 1-3) des vorgenannten Dokuments nennt zahlreiche feste Säuren, aus welchen der Fachmann, gegebenenfalls nach Bestimmung des H₀-Werts, den geeigneten Katalysator auswählen kann. Es eignen sich vorzugsweise (i) natürliche oder synthetische silikatische Stoffe wie insbesondere Mordenit, Montmorillonit und saure Zeolithe, wie beispielsweise HZSM-5, MCM-22, Zeolith Beta; (ii) mit ein-, zwei- oder mehrbasigen anorganischen Säuren insbesondere Phosphorsäure, Schwefelsäure oder sauren Salzen anorganischer Säuren belegt Trägermaterialien, wie oxidische oder silikalitische Stoffe, beispielsweise Aluminiumoxid, Titanoxid, Siliziumoxid, Zirkonoxid oder deren Mischungen; (iii) Oxide und Mischoxide, wie beispielsweise Aluminiumoxide, Zinkoxid-Aluminiumoxid-Mischungen oder Heteropolysäuren.

Das Verfahren lässt sich in der Flüssig- oder in der Gasphase durchführen. In beiden Ausführungsformen können im Prinzip die gleichen sauren Katalysatoren eingesetzt werden. Es hat sich aber gezeigt, dass bestimmte Katalysatoren vorzugsweise für die Gasphase und andere vorzugsweise für die Flüssigphase geeignet sind.

So sind in der Flüssigphase bevorzugt sauren Zeolithe wegen ihres H₀-Wertes von kleiner -3 einsetzbar. In der Gasphase unterliegen sie dagegen einer schnelleren Desaktivierung, was die Raum-Zeit-Ausbeute verringert. Oxide und Mischoxide hingegen liefern in der Gasphase die besseren Ausbeuten.

Bei der Durchführung der Glycerinumsetzung zu MMP in der Flüssigphase können auch entsprechend saure homogene Katalysatoren, die im Reaktionsgemisch löslich sind, verwendet werden. Die homogenen Katalysatoren können dabei alleine oder in Kombination mit einem hier beschriebenen heterogenen Katalysator eingesetzt werden.

In einer weiteren Ausführungsform der Erfindung wird Methylmercaptan nicht sofort zu Reaktionsbeginn dem Reaktionsgemisch zugeführt, sondern erst eingespeist, nachdem sich reaktive Intermediate bei den oben angegebenen Bedingungen zumindest aus Teilmengen von Glycerin gebildet haben. Die reaktiven Intermediate bilden durch Weiterreaktion nach Zusatz von Methylmercaptan MMP. Diese Vorgehensweise erleichtert einerseits die Umsetzung von Glycerin zu dehydratisierten reaktiven Intermediaten und vermindert unselektive Reaktionen zwischen Glycerin und Methylmercaptan. Dadurch kann die Ausbeute zu MMP verbessert werden. Gleichzeitig wird durch diese Reaktionsführung weiterhin die nach dem Stand der Technik aufwendige Auftrennung von Acrolein vermieden und die Reaktion zu MMP in einem Reaktor durchgeführt.

Dies kann einerseits in einer diskontinuierlichen Fahrweise erfolgen, wobei das Methylmercaptan nach einer gewissen Reaktionszeit dem Reaktionsgemisch zugesetzt wird. Geeignet wäre dafür beispielsweise die Durchführung im Rührkessel. Weiterhin ist auch eine halbkontinuierliche Fahrweise möglich, indem die Glycerinlösung und der Katalysator vorgelegt werden und das Methylmercaptan kontinuierlich zudosiert wird.

Andererseits kann Methylmercaptan auch erst an einem vom Reaktoreingang in Strömungsrichtung entfernten Ort oder in einem weiteren Reaktorabschnitt eingespeist werden. Bis die Glycerinlösung und der Katalysator diesen Ort erreichen, wurde bereits ein Teil des Glycerins zu reaktiven Intermediaten umgesetzt. Diese Fahrweise kann technisch beispielsweise in einem Hordenreaktor mit Zwischeneinspeisung, einer Rührkesselkaskade oder einem Strömungsrohr realisiert werden.

Ein weiterer Vorteil der verzögerten bzw. späteren Zugabe von zumindest einem größeren Teilen des Methylmercaptans besteht darin, dass die Ausbeute an MMP gesteigert werden kann, weil ein anhand dem Reaktionsverhalten der Reaktanden optimiertes Temperaturprofil bzw. Temperaturprogramm eingestellt werden kann. So erfordert die Aktivierung von Glycerin und dessen erster Dehydratisierungsschritt eine sehr hohe Aktivierungsenergie und somit hohe Reaktionstemperaturen zur schnellen Herstellung mit hohen Raum-Zeit-Ausbeuten. MMP tendiert hingegen bei Temperaturen höher als ca. 100 bis 150 °C zu unselektiven Weiterreaktionen. Somit stellt die Durchführung der Umsetzung von Glycerin zu MMP mittels dem Durchlaufen sinkender Reaktionstemperaturen eine bevorzugte Ausführungsform dar.

### Beispiele

### Beispiel 1

In einem Autoklav wurden in 144 g Methanol 36 g Glycerin und 19,5 g Methylmercaptan gelöst. Zu diesem Gemisch wurden 3,8 g Zeolith HZSM-5, Modul 28 (H₀ < -8,2) hinzugegeben. Der Zeolith wurde vor dem Einsatz im Autoklav 2 h bei 150 °C und 4 h bei 500 °C in Luft im Trockenofen kalziniert. Anschließend wurde das Gemisch im Autoklav unter Rühren auf 300 °C aufgeheizt, Dabei stellte sich ein Druck von 61 bar ein. Nach einer Stunde wurde eine Probe aus dem Gemisch entnommen und mittels Gaschromatographie analysiert. Unter Berücksichtigung des Lösungsmittelanteils betrug der Gehalt an MMP 6,0 Gew.-%. Es war keines der Neben- bzw. Zwischenprodukte Acrolein und Allylalkohol detektierbar.

### Beispiel 2

In einem Autoklav wurden in 80 g Methanol 36 g Glycerin gelöst. Zu diesem Gemisch wurden 3,8 g Zeolith Ammonium Beta CP 814E (H₀ < -3,0) der Fa. Zeolyst International hinzugegeben. Der Zeolith wurde vor dem Einsatz im Autoklav 2 h bei 150 °C und 4 h bei 500 °C in Luft im Trockenofen kalziniert.. Das Gemisch wurde dann innerhalb von 4 h aufgeheizt und bei 300 °C und 40 bar gerührt. Nach dem Abkühlen auf Raumtemperatur erfolgte die Zugabe von 19,5 g Methylmercaptan und 68 g Methanol. Anschließend wurde dieses neue Gemisch im Autoklav unter Rühren auf 100 °C aufgeheizt. Dabei stellte sich ein Druck von 3 bar ein. Nach 30 min wurde eine Probe aus dem Gemisch entnommen und mittels Gaschromatographie analysiert. Unter Berücksichtigung des Lösungsmittelanteils betrug der Gehalt an MMP 0,8 Gew.-%.

## Patentansprüche

1. Verfahren zur Herstellung von Verbindungen der allgemeinen Formel 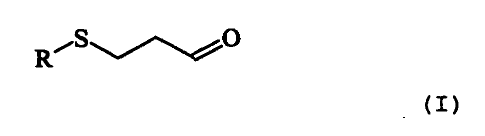 in der bedeuten
R: H, C₁ bis C₃-Alkyl, durch die Umsetzung von Glycerin oder einer Verbindung, aus der Glycerin freigesetzt wird, mit einer Verbindung der allgmeinen Formel
R-SH (II)
in der bedeutet
R: H, C₁ bis C₃-Alkyl
oder Verbindungen, aus denen diese (II) hergestellt wird,
in Gegenwart eines sauren Feststoffkatalysators, dessen H₀-Wert (Hammet'sche Säurefunktion) kleiner +2 ist.

2. Verfahren gemäss Anspruch 1, **dadurch gekennzeichnet, dass** R in Formel II Methyl entspricht und MMP hergestellt wird.

3. Verfahren gemäss den Ansprüchen 1 bis 2, **dadurch gekennzeichnet, dass** das molare Verhältnis von Glycerin zu Methylmercaptan zwischen 0,2 und 50 beträgt.

4. Verfahren gemäss den Ansprüchen 1 bis 3, **dadurch gekennzeichnet, dass** ein Lösungs- oder Verdünnungsmittel eingesetzt wird.

5. Verfahren gemäss den Ansprüchen 1 bis 4, **dadurch gekennzeichnet, dass** das Glycerin im Reaktionsgemisch in verdünnter Form mit einem Gehalt von 1 bis 100 Ges.-%, bevorzugt zwischen 1 und 70 Gew.-% und insbesondere zwischen 5 und 40 Gew.-%, bezogen auf das Lösungs- oder Verdünnungsmittel, eingesetzt wird.

6. Verfahren gemäss den Ansprüchen 4 oder 5, **dadurch gekennzeichnet, dass** als Lösungs- oder Verdünnungsmittel MMP eingesetzt wird.

7. Verfahren gemäss den Ansprüchen 4 oder 5, **dadurch gekennzeichnet, dass** als Lösungs- oder Verdünnungsmittel Wasser eingesetzt wird.

8. Verfahren gemäss den Ansprüchen 4 oder 5, **dadurch gekennzeichnet, dass** als Lösungs- oder Verdünnungsmittel Methanol eingesetzt wird.

9. Verfahren gemäss den Ansprüchen 1 bis 8, **dadurch gekennzeichnet, dass** das Verfahren in der Flüssigphase erfolgt.

10. Verfahren gemäss Anspruch 9, **dadurch gekennzeichnet, dass** dies bei Drücken zwischen 1 und 300 bar sowie bei Temperaturen zwischen 20 und 500 °C erfolgt.

11. Verfahren gemäss Anspruch 9, **dadurch gekennzeichnet, dass** als Katalysator ein saurer Zeolith verwendet wird.

12. Verfahren gemäss den Ansprüchen 1 bis 2 und 3 bis 8, **dadurch gekennzeichnet, dass** die Verfahren in der Flüssigphase in Gegenwart eines homogenen Katalysators bei Temperaturen zwischen -10 und 500 °C sowie Drücken zwischen 1 und 300 bar erfolgt.

13. Verfahren gemäss Anspruch 1 bis 8, **dadurch gekennzeichnet, dass** die Verfahren in der Gasphase erfolgt.

14. Verfahren gemäss Anspruch 13, **dadurch gekennzeichnet, dass** dies bei Drücken zwischen 1 und 100 bar sowie bei Temperaturen zwischen 200 und 550 °C erfolgt.

15. Verfahren gemäss Anspruch 13 oder 14, **dadurch gekennzeichnet, dass** ein Katalysator mit einem H₀-Wert kleiner -3 verwendet wird.

16. Verfahren gemäss den Ansprüchen 1 bis 15, **dadurch gekennzeichnet, dass** Methylmercaptan in einer Menge > 0 % dem Reaktionsgemisch nach der Umsetzung von Teilmengen des Glycerins zu Intermediaten zugegeben wird oder dass ein Glycerin und zu Intermediaten umgesetztes Glycerin enthaltendes Reaktionsgemisch zu Methylmercaptan zudosiert wird.

17. Verfahren gemäss Anspruch 16, **dadurch gekennzeichnet, dass** die Umsetzung in mindestens zwei räumlich getrennten Zonen abläuft, wobei Glycerin in der ersten Reaktionszone zumindest teilweise umgesetzt wird und Methylmercaptan in die darauffolgende Reaktionszone eingespeist wird.

18. Verfahren gemäss Anspruch 17, **dadurch gekennzeichnet, dass** in beiden Reaktionszonen unterschiedliche Temperaturen vorliegen, wobei die Temperatur in der ersten Reaktionszone mit 150 bis 400 °C höher ist als in der darauffolgenden Zone, in der die Temperaturen zwischen 0 bis 100 °C liegen.

19. Verfahren gemäss Anspruch 17 und 18, **dadurch gekennzeichnet, dass** Methylmercaptan oder dessen größerer Anteil (> 50 %) erst nach dem Abkühlen des Glycerins oder daraus entstandene Intermediate enthaltenden Reaktionsgemisches auf 20 bis 150 °C eingespeist wird.

## Claims

1. Process for preparing compounds of the general formula 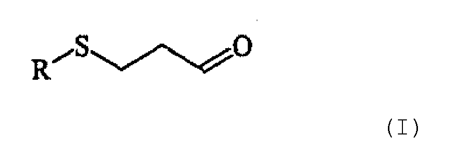 in which
R is H, C₁ to C₃-alkyl, by the reaction of glycerol or of a compound from which glycerol is released with a compound of the general formula
R-SH (II)
in which
R is H, C₁ to C₃-alkyl
or compounds from which this (II) is prepared,
in the presence of an acidic solid catalyst whose H₀ value (Hammett acidity function) is less than +2.

2. Process according to Claim 1, **characterized in that** R in formula II is methyl, and MMP is prepared.

3. Process according to Claim 1 or 2, **characterized in that** the molar ratio of glycerol to methyl mercaptan is between 0.2 and 50.

4. Process according to Claims 1 to 3, **characterized in that** a solvent or diluent is used.

5. Process according to Claims 1 to 4, **characterized in that** the glycerol is used in the reaction mixture in diluted form with a content of 1 to 100% by weight, preferably between 1 and 70% by weight and especially between 5 and 40% by weight, based on the solvent or diluent.

6. Process according to Claim 4 or 5, **characterized in that** the solvent or diluent used is MMP.

7. Process according to Claim 4 or 5, **characterized in that** the solvent or diluent used is water.

8. Process according to Claim 4 or 5, **characterized in that** the solvent or diluent used is methanol.

9. Process according to Claims 1 to 8, **characterized in that** the process proceeds in the liquid phase.

10. Process according to Claim 9, **characterized in that** it proceeds at pressures between 1 and 300 bar and at temperatures between 20 and 500°C.

11. Process according to Claim 9, **characterized in that** the catalyst used is an acidic zeolite.

12. Process according to Claims 1 to 2 and 3 to 8, **characterized in that** the process proceeds in the liquid phase in the presence of a homogeneous catalyst at temperatures between -10 and 500°C and pressures between 1 and 300 bar.

13. Process according to Claims 1 to 8, **characterized in that** the process proceeds in the gas phase.

14. Process according to Claim 13, **characterized in that** it proceeds at pressures between 1 and 100 bar and at temperatures between 200 and 550°C.

15. Process according to Claim 13 or 14, **characterized in that** a catalyst with an H₀ value of less than -3 is used.

16. Process according to Claims 1 to 15, **characterized in that** methyl mercaptan is added in an amount of > 0% to the reaction mixture after the conversion of portions of the glycerol to intermediates, or in that a reaction mixture comprising glycerol and glycerol converted to intermediates is metered into methyl mercaptan.

17. Process according to Claim 16, **characterized in that** the conversion proceeds in at least two spatially separate zones, glycerol being converted at least partly in the first reaction zone and methyl mercaptan being fed into the downstream reaction zone.

18. Process according to Claim 17, **characterized in that** there are different temperatures in the two reaction zones, the temperature in the first reaction zone being 150 to 400°C higher than in the downstream zone in which the temperatures are between 0 and 100°C.

19. Process according to Claim 17 or 18, **characterized in that** methyl mercaptan or the majority thereof (> 50%) is fed in only after the reaction mixture comprising glycerol or intermediates formed therefrom has been cooled to 20 to 150°C.

## Revendications

1. Procédé pour la préparation de composés de formule générale 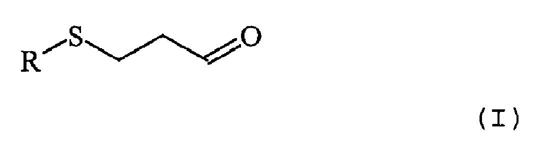 dans laquelle
R représente H, un groupe alkyle en C₁-C₃, par la mise en réaction de glycérol ou d'un composé à partir duquel est libéré du glycérol, avec un composé de formule générale
R-SH (II)
dans laquelle
R représente H, un groupe alkyle en C₁-C₃,
ou des composés à partir desquels est préparé ce composé (II),
en présence d'un catalyseur solide acide dont l'indice H₀ (fonction acide de Hammet) est inférieur à +2.

2. Procédé selon la revendication 1, **caractérisé en ce que** R dans la formule II correspond au groupe méthyle et on prépare le MMP [3-(méthylthio)-propanal].

3. Procédé selon les revendications 1 et 2, **caractérisé en ce que** le rapport molaire du glycérol au méthylmercaptan est compris entre 0,2 et 50.

4. Procédé selon les revendications 1 à 3, **caractérisé en ce qu'**on utilise un solvant ou diluant.

5. Procédé selon les revendications 1 à 4, **caractérisé en ce que** le glycérol est introduit dans le mélange réactionnel sous forme diluée, à une concentration de 1 à 100 % en poids, de préférence comprise entre 1 et 70 % en poids et en particulier entre 5 et 40 % en poids, par rapport au solvant ou diluant.

6. Procédé selon la revendication 4 ou 5, **caractérisé en ce que** le MMP est utilisé comme solvant ou diluant.

7. Procédé selon la revendication 4 ou 5, **caractérisé en ce qu'**on utilise l'eau comme solvant ou diluant.

8. Procédé selon la revendication 4 ou 5, **caractérisé en ce qu'**on utilise le méthanol comme solvant ou diluant.

9. Procédé selon les revendications 1 à 8, **caractérisé en ce que** le procédé est effectué en phase liquide.

10. Procédé selon la revendication 9, **caractérisé en ce qu'**il est effectué sous des pressions comprises entre 1 et 300 bars ainsi qu'à des températures comprises entre 20 et 500 °C.

11. Procédé selon la revendication 9, **caractérisé en ce qu'**on utilise comme catalyseur une zéolithe acide.

12. Procédé selon les revendications 1 et 2 et 3 à 8, **caractérisé en ce que** le procédé est effectué en phase liquide en présence d'un catalyseur homogène, à des températures comprises entre -10 et 500 °C ainsi que sous des pressions comprises entre 1 et 300 bars.

13. Procédé selon les revendications 1 à 8, **caractérisé en ce que** le procédé est effectué en phase gazeuse.

14. Procédé selon la revendication 13, **caractérisé en ce qu'**il est effectué sous des pressions comprises entre 1 et 100 bars ainsi qu'à des températures comprises entre 200 et 550 °C.

15. Procédé selon la revendication 13 ou 14, **caractérisé en ce qu'**on utilise un catalyseur ayant un indice H₀ inférieur à -3.

16. Procédé selon les revendications 1 à 15, **caractérisé en ce que** le méthylmercaptan est ajouté par addition dosée au mélange réactionnel en une quantité > 0 % après la conversion de quantités partielles du glycérol en produits intermédiaires ou **en ce qu'**on ajoute par addition dosée au méthylmercaptan un mélange réactionnel contenant du glycérol et du glycérol converti en produits intermédiaires.

17. Procédé selon la revendication 16, **caractérisé en ce que** la réaction se déroule dans au moins deux zones séparées dans l'espace, le glycérol dans la première zone de réaction étant au moins partiellement converti et le méthylmercaptan étant introduit dans la zone de réaction suivante.

18. Procédé selon la revendication 17, **caractérisé en ce que** des températures différentes sont présentes dans les deux zones de réaction, la température dans la première zone de réaction étant supérieure de 150 à 400 °C à celle dans la zone suivante, dans laquelle les températures sont comprises entre 0 et 100 °C.

19. Procédé selon les revendications 17 et 18, **caractérisé en ce qu'**on n'introduit le méthylmercaptan ou sa majeure partie (> 50 %) qu'après le refroidissement jusqu'à une température de 20 à 150 °C du glycérol ou du mélange réactionnel contenant des produits intermédiaires en résultant.
